# EUROPEAN PATENT APPLICATION

(11) **EP 4 503 038 A1**
(43) Date of publication of application: **05.02.2025**
(21) Application number: 23780146.9
(22) Date of filing: 24.03.2023
(51) Int. Cl.: G16B 40/00, C12M 1/00, C12M 1/34, C12N 15/11, C12Q 1/6844, C12Q 1/6869, G01N 33/50, G06N 20/00, G16B 30/00

(54) **LEARNING SYSTEM, DECISION SYSTEM, AND PREDICTION SYSTEM, AND LEARNING METHOD, DECISION METHOD, AND PREDICTION METHOD**

(30) Priority: 30.03.2022 JP 2022056626
(71) Applicant: FUJIFILM Corporation, Tokyo 106-8620 (JP)
(72) Inventor: SINGH, Janmajay, Ashigarakami-gun, Kanagawa 258-8538 (JP)
(74) Representative: Dehns Germany Partnerschaft mbB
(86) International application number: PCT/JP2023/011772
(87) International publication number: WO 2023/190136

(57) **Abstract**

An embodiment of the present invention provides a learning system, a determination system, a prediction system, a learning method, a determination method, and a prediction method. In DNA methylation measurement, there are a problem of incomplete bisulfite conversion (problem 1), a problem of the occurrence of bias in a case where a plurality of different biomarker sequences/genes are amplified together (problem 2), and a problem in which the degree of excessive amplification of an unmethylated signal depends on a gene sequence and a chemical substance used for the measurement (problem 3). An aspect of the present invention provides a system that learns measurement error characteristics in the presence of the three problems and reflects the learned error characteristics in a biomarker selection criterion and a method corresponding to the system. Addressing the problem of evaluating the measurement error characteristics for DNA methylation under the presence of combinations of the problems 1 to 3 forms the major novelty of the present invention.

## Description

### BACKGROUND OF THE INVENTION

### 1. Field of the Invention

The present invention relates to a technique that measures a value of a biomarker.

### 2. Description of the Related Art

It is known that a phenomenon called "methylation" occurs in deoxyribonucleic acid (DNA). The methylation means a modification by chemical bonding of a methyl molecule to cytosine. This cytosine (C) constitutes four essential nucleic-acid bases constituting DNA together with guanine (G), adenine (A), and thymine (T). Any sequence of the nucleic-acid bases is referred to as a "nucleotide sequence", and the nucleotide sequence that codes for important information, such as a protein, is referred to as a "genome sequence" or a "gene".

In humans, the methylation is particularly common at a site (called a "CpG site") where cytosine is followed by guanine on a DNA strand. A methylated state affects the activation or suppression of the gene. The methylated state of the CpG site of a certain gene forms an important biomarker for many diseases. In general, data obtained from a combination of several biomarker candidate sequences is used to create a quantitative model for disease diagnosis. Therefore, it is important to measure DNA methylation of the biomarker.

An error is added to data in a DNA measurement process, which affects the reliability of any estimation/prediction. In the research according to the related art for optimizing the selection of the biomarker, a slight error is assumed in the measurement process, and focus is placed on only the prediction value of available data. As an example of this method, a feature selection algorithm is known that determines whether or not to use a biomarker sequence as a feature for classification on the basis of an output signal from a quantitative model (such as the performance of an artificial intelligence classifier).

For this technique according to the related art, for example, JP2017-523437A discloses a technique that selects a biomarker set from representative biomarker data and evaluates the biomarker set. In addition, "Measuring and Mitigating PCR Bias in Microbiome Data", Justin D. Silverman et al., [Searched on March 22, 2022], Internet (https://www.biorxiv.org/content/10.1101/604025v1) discloses the measurement and mitigation of polymerase chain reaction (PCR) bias.

### SUMMARY OF THE INVENTION

In the next section, the previous studies that have attempted to learn the measurement error characteristics of the biomarker sequence will be discussed in detail. These prior studies and the problems related to them will be discussed, and the details of each stage will be described.

### [Measurement of DNA Methylation]

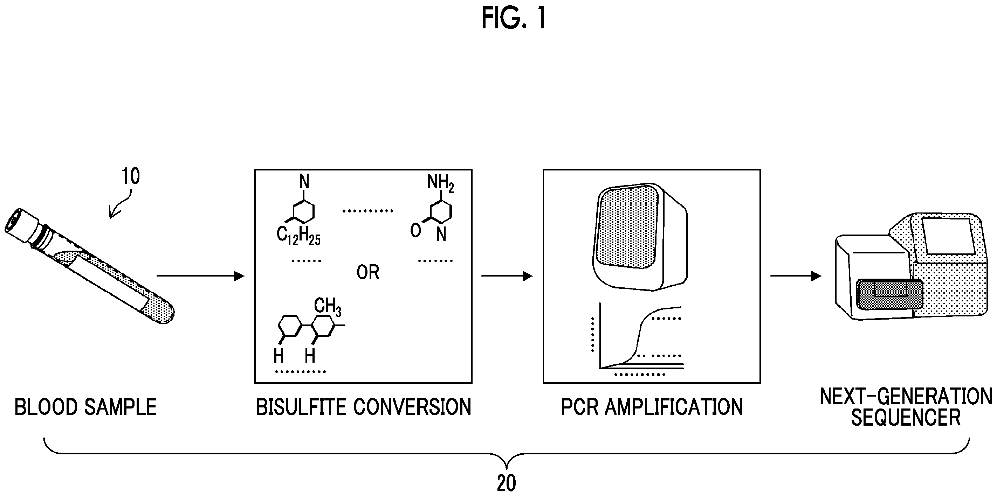

An outline of methylation measurement is illustrated in Fig. 1. In the measurement of methylation, a blood sample 10 is subjected to bisulfite conversion, a gene/signal is amplified by a PCR device, and methylation is measured by a next-generation sequencer or the like. The series of measurement procedures constitutes a wet experiment protocol 20.

### [STEP 1: Bisulfite Conversion]

An additional step of the bisulfite conversion is used in order to distinguish between Cm (methylated cytosine) and Cu (unmethylated cytosine). In the bisulfite conversion, Cu is converted into uracil (U), and Cm remains without any change. In a case where the converted sample is sequenced, Cm is read as C (cytosine), and uracil is read as thymine. Therefore, it is possible to distinguish the methylated state of cytosine.

### [Problem 1: Problem in Bisulfite Conversion]

An ideal result of this procedure is that Cu is converted into uracil by 100%, and Cm is not converted into uracil at all (the conversion is 0% and Cm remains without any change). However, due to the nature of a chemical reaction, the degree of success (or failure) of the conversion is probabilistic, and it is difficult to carry out quantitative research. Hereinafter, this incompleteness of the bisulfite conversion is referred to as "problem 1".

### [STEP 2: PCR Amplification]

This stage can be understood as a signal amplification stage of measurement. As a standard (that is, not for methylation but for bisulfite conversion), each "signal" is a gene or a sequence of interest. In raw data, the number of sequences is extremely small. Therefore, a derived signal is weak. Therefore, it is considered that the original sequence is copied multiple times to increase the number of sequences and to amplify the signal. For example, it is assumed that the signal intensity of gene 1 before PCR is referred to as G1_pre and the signal intensity of gene 1 after PCR is referred to as G1_post. Further, in practice, focus is placed on the simultaneous amplification of many genes/signals. Therefore, for gene 2, G2_pre and G2_post are defined in the same manner as the gene 1.

Now, in a case where the above-mentioned STEP 1 is performed first, two signals are obtained from only one gene. For example, the gene 1 has some sequences that have unmethylated CpG and that are converted into another sequence including uracil. Similarly, the sequence in which CpG is methylated is not converted. This is common and is observed in a DNA mixture of the liver and the stomach. In this mixture, there is a possibility that an important gene in the liver will not be methylated in liver cells, but will be methylated in gastric cells (thus, will be suppressed). Therefore, for the gene 1, it is assumed that the intensity of the signal before PCR and the intensity of the signal after PCR are G1_U_Pre and G1_U_post (in a case where the gene 1 is not methylated) and G1_M_Pre and G1_M_post (in a case where the gene 1 is methylated), and the decoded sequences are G1_M_Pre and G1_M_post.

### [Problem 2: PCR Bias in Single Bisulfite Protocol]

Even in a case where the signal of the same gene is amplified, the bisulfite conversion results in two signal types. Therefore, G1_U_post/G1_U_pre = G1_M_post/G1_M_pre is not established. It is known that, even in a case where G1_U_pre = G1_M_pre is established, G1_U_post/G1_U_pre > G1_M_post/G1_M_pre is satisfied after amplification (that is, the unmethylated gene is excessively amplified with respect to the methylated gene). However, the degree of excessive amplification of the unmethylated signal depends on the gene sequence and the chemical substance used for the measurement. Hereinafter, this problem is referred to as "problem 2".

### [Problem 3: Problem in PCR amplification]

An ideal result of PCR is G1_post/G1_pre = G2_post/G2_pre. However, in practice, some gene sequences are easier to measure than others, and this equivalence is not established. This bias that occurs in a case where several different biomarker sequences/genes are amplified together is referred to as a "PCR bias" in a multiplex protocol (hereinafter, referred to as "problem 3").

### [Response in Related Art]

The response to the above-described problems 1 to 3 in the related art will be described. In the related art, for the problem 1, quantitative studies often do not require extreme precision. Therefore, the degree of success of bisulfite conversion has not been considered. In addition, for the problem 3, in the previous microbiological research, the effect of PCR has been considered multiplicatively. That is, in the related art, it has been considered that, in a case where the signal intensity of gene 1 after one cycle of PCR is j, the signal intensity after two cycles is j² and the signal intensity after x cycles is similarly j^{x}. This assumption was used to model the PCR as a logarithmic linear process using a multinomial logistic-general linear model. Other covariates, such as "batch effect" (PCR for specimens that show slightly different bias characteristics for each batch), were also included in a probabilistic manner. The model is "trained" using the generated calibration data and then used to correct the PCR bias.

In addition, for the problem 2, since the characterization of the measurement error and the bias in single protocol settings is simpler, linear regression is performed in some PCR data to find the degree of the bias. After calculating a linear regression estimation amount, the bias can be corrected using this equation.

The importance of the accurate measurement of DNA methylation has already been described. In an application field, such as disease diagnosis, it is not uncommon to use data of a plurality of biomarker sequences as an input to a quantitative model. In a case where a measurement process for simultaneously measuring metric values of a plurality of biomarkers is designed, the problems 1, 2, and 3 are combined, and all of them are problematic. Therefore, it is very difficult to quantify the error and to characterize the learning error. In the present invention, a system is examined that learns measurement error characteristics in the presence of three problems and reflects the learned error characteristics in a biomarker selection criterion. Addressing the problem of measurement error characteristic evaluation for DNA methylation under the presence of combinations of the problems forms the main novelty of the present invention.

The present invention is particularly important in a case where simultaneous and extremely accurate measurement of DNA methylation from a plurality of genes is required as in liquid biopsy. In particular, it is known that, for accurate identification of a disease, such as cancer, certain cancer cell genes show higher methylation than the same genes in healthy cells. In this case, the problem 2 means that the measurement underestimates a true methylation ratio from a mixture of cancer and normal DNA (negative bias). The problems 1 and 3 further increase the degree of underestimation.

The present invention has been made in view of the above circumstances, and an embodiment of the present invention provides a learning system and a learning method that learn measurement error characteristics of a biomarker sequence. In addition, another embodiment of the present invention provides a determination system and a determination method that reflect the learned error characteristics to determine a sequence set and a prediction system and a prediction method that predict measurement error characteristics of a gene sequence using data obtained by the learning system or the learning method.

According to a first aspect of the present invention, there is provided a learning system that learns a relationship between a measurement protocol variable and an error characteristic occurring as a result of a biomarker sequence. The learning system comprises a processor configured to: input calibration data that is designed such that appropriate data is capable of being acquired for an important variable; and learn a characteristic of an error distribution over each measurement protocol for the important variable, using a probability model. The probability model includes a first parameter that is initialized with an appropriately selected prior parameter in order to model an error of bisulfite conversion, a second parameter that is initialized with an appropriately selected prior parameter in order to model interdependency of amplification of the biomarker sequence, and a third parameter that is initialized with an appropriately selected prior parameter in order to model a bias of an entire PCR. The learning system according to the first aspect is a system that learns a relationship between the measurement protocol variable and the error characteristic occurring as the result of the biomarker sequence (defined as a template-to-product ratio).

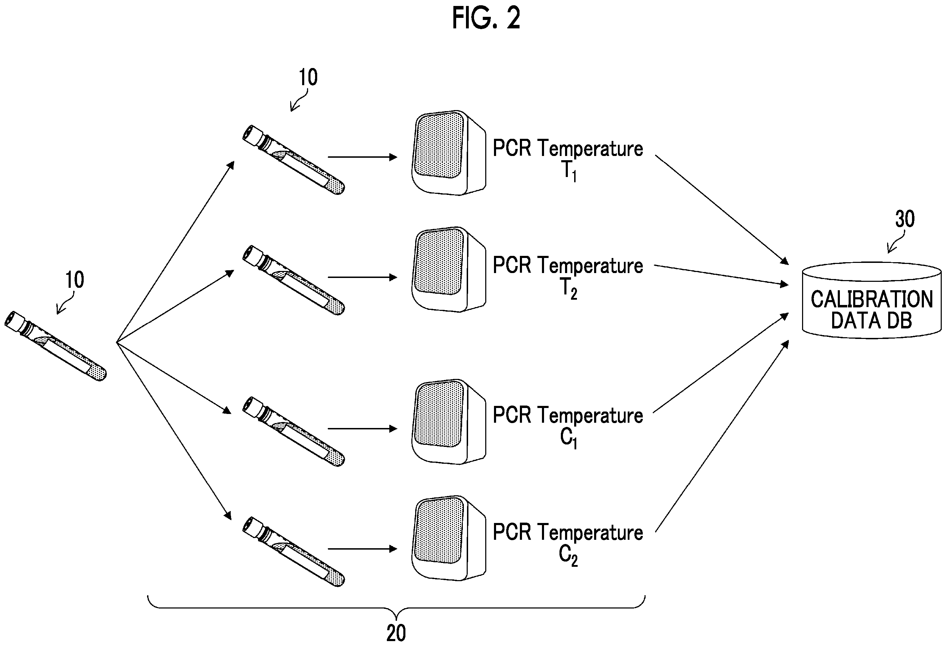

In the first aspect and each of the following aspects, the "important variable" is a variable that is known to affect signal amplification performance by an expert in a laboratory, and a PCR device is adjusted for this variable. For example, a PCR temperature and the number of PCR cycles illustrated in Fig. 2 which will be described below are examples of the "important variable". In a case where the temperature is too high, DNA is decomposed, and a reaction necessary for replicating a target gene sequence does not occur. In addition, the same parameters may be used as the "appropriately selected prior parameters" for the first to third parameters. Further, for the "input of the calibration data", for example, in a case of the PCR temperature, it is necessary to appropriately display the PCR temperature in a temperature range used in a normal PCR.

According to a second aspect, in the learning system according to the first aspect, the second parameter may be a parameter that is obtained by separately acquiring counts of methylated sequences and unmethylated sequences of genes after the bisulfite conversion and modeling the acquired counts with a multinomial distribution capable of separately determining a prior variable for each of the methylated sequences and the unmethylated sequences. The second aspect defines a specific aspect of the second parameter for responding to the above-mentioned problem 2 and can model and correct the error of the bisulfite conversion to correctly evaluate the methylation of the biomarker sequence. In the second aspect, it is possible to select a better prior variable from empirical data analysis. In addition, the counts acquired in the second aspect can be modeled on the basis of a factor such as a guanine-cytosine ratio (GC ratio) of a base sequence.

According to a third aspect, in the learning system according to the first or second aspect, the third parameter may be a parameter subjected to a configuration data constraint in which a sum of individual counts calculated by a multinomial distribution follows a Gaussian distribution in a case where a plurality of sequences are simultaneously amplified using a universal primer. The third aspect defines a specific aspect of the second parameter for responding to the problem 3. In a case where the number of biomarkers is large and modeling parameters are simplified such that the configuration data constraint can be calculated, the sum of the counts in a plurality of dispersion counts follows the Gaussian distribution. In addition, in a case where a plurality of sequences are amplified at the same time, the count values of the individual markers are not independent, and the amplification is performed such that the sum of the counts is almost constant. Therefore, the modeling using the multinomial distribution is suitable. Further, in methylation measurement accompanied by bisulfite conversion, since each marker has two states of a methylated state and an unmethylated state, modeling for a count value of the number of markers × 2 is performed.

According to a fourth aspect of the present invention, there is provided a determination system comprising a processor configured to: input a nucleotide sequence of a biomarker sequence of interest and measurement protocol information used in a multiplex panel; input the learned error characteristic and metadata associated with the error characteristic from the learning system according to any one of the first to third aspects; output a first score for a set of possible biomarker sequences using the input nucleotide sequence, measurement protocol information, learned error characteristic, and metadata, according to a predetermined criterion; and determine a biomarker sequence set in consideration of a value of the first score for each set. In the determination system according to the fourth aspect, the output from the system according to the first aspect is used to determine whether or not to use the biomarker sequence in the multiplex panel. The first score is a score derived from measurement accuracy and is a "low error score" that is higher as the measurement error is smaller.

According to a fifth aspect, in the determination system according to the fourth aspect, the processor may be configured to: input a second score for each biomarker sequence to be determined; and optimize a balance between the first score and the second score in consideration of the first score for each biomarker sequence in the biomarker sequence set to select a best subset of the multiplex panel. In the determination system according to the fifth aspect, the fourth aspect is enhanced by considering the final goal of the multiplex panel to enable more balanced selection of the biomarker sequence. The second score is, for example, a score (degree-of-association score) that is higher as the degree of association with the disease to be predicted is larger. In addition, the "balance between the first score and the second score" can be optimized, for example, by calculating a third score defined as the arithmetic mean or geometric mean of the first score and the second score and maximizing the third score.

According to a sixth aspect of the present invention, there is provided a prediction system that predicts a measurement error characteristic of a gene sequence. The prediction system comprises a processor configured to: input a nucleotide sequence of a biomarker sequence of interest and measurement protocol information used in a multiplex panel; input the learned error characteristic and metadata associated with the error characteristic from the learning system according to any one of the first to third aspects; calculate a similarity degree between a biomarker sequence previously included in calibration data and a new biomarker sequence, using a measurement criterion for calculating a measure of similarity between two gene sequences; and predict an error characteristic in a case of measuring a biomarker sequence that is not included in the calibration data, using the calculated similarity degree in combination with other related inputs and the learned error characteristic. The prediction system according to the sixth aspect enables the learning system according to the first to third aspects to be used for the biomarker sequence that is not included in the calibration data.

In addition, in the sixth aspect, the "other related inputs" mean, for example, metadata corresponding to the biomarker sequence. For example, in a case in which the gene type is a "promoter or enhancer", the CpG type is an "island, a shore, or a shelf", and the abundance of CG is "high or low", a combination of these information items (an example of metadata) for a certain biomarker sequence G1 can be represented as a vector "promoter, island, low".

According to a seventh aspect, in the prediction system according to the sixth aspect, the processor may be configured to: acquire a biomarker sequence that is most similar to the biomarker sequence not included in the calibration data and that has been available in the calibration data, using the predicted error characteristic; and reflect information of the acquired biomarker sequence in the determination of the biomarker sequence set in the determination system according to the fourth or fifth aspect. The seventh aspect makes it possible to use the biomarker sequence that is not included in the calibration data in the selection of the biomarker sequence set, using the determination system according to the fourth or fifth aspect.

According to an eighth aspect of the present invention, there is provided a learning method executed by a learning system that includes a processor and learns a relationship between a measurement protocol variable and an error characteristic occurring as a result of a biomarker sequence. The learning method comprises: causing the processor to input calibration data that is designed such that appropriate data is capable of being acquired for an important variable (calibration data input step) and to learn a characteristic of an error distribution over each measurement protocol for the important variable, using a probability model (learning step). The probability model includes a first parameter that is initialized with an appropriately selected prior parameter in order to model an error of bisulfite conversion, a second parameter that is initialized with an appropriately selected prior parameter in order to model interdependency of amplification of the biomarker sequence, and a third parameter that is initialized with an appropriately selected prior parameter in order to model a bias of an entire PCR. The eighth aspect defines the learning method corresponding to the first aspect described above.

According to a ninth aspect, in the learning method according to the eighth aspect, the second parameter may be a parameter that is obtained by separately acquiring counts of methylated sequences and unmethylated sequences of genes after the bisulfite conversion and modeling the acquired counts with a multinomial distribution capable of separately determining a prior variable for each of the methylated sequences and the unmethylated sequences. The ninth aspect defines the learning method corresponding to the second aspect.

According to a tenth aspect, in the learning method according to the eighth or ninth aspect, the third parameter may be a parameter subjected to a configuration data constraint in which a sum of individual counts calculated by a multinomial distribution follows a Gaussian distribution in a case where a plurality of sequences are simultaneously amplified using a universal primer. The tenth aspect defines the learning method corresponding to the third aspect.

According to an eleventh aspect of the present invention, there is provided a determination method executed by a determination system including a processor. The determination method comprises: causing the processor to input a nucleotide sequence of a biomarker sequence of interest and measurement protocol information used in a multiplex panel (sequence information input step), to input the learned error characteristic obtained as a result of the learning method according to any one of the eighth to tenth aspects and metadata associated with the error characteristic (learning result input step), to output a first score for a set of possible biomarker sequences using the input nucleotide sequence, measurement protocol information, learned error characteristic, and metadata, according to a predetermined criterion (score output step), and to determine a biomarker sequence set in consideration of a value of the first score for each set (sequence set determination step). The eleventh aspect defines the determination method corresponding to the fourth aspect.

According to a twelfth aspect, the determination method according to the eleventh aspect may further comprise: causing the processor to input a second score for each biomarker sequence to be determined (score input step) and to optimize a balance between the first score and the second score in consideration of the first score for each biomarker sequence in the biomarker sequence set to select a best subset of the multiplex panel (subset selection step). The twelfth aspect defines the determination method corresponding to the fifth aspect.

According to a thirteenth aspect of the present invention, there is provided a prediction method executed by a prediction system that includes a processor and that predicts a measurement error characteristic of a gene sequence. The prediction method comprises: causing the processor to input a nucleotide sequence of a biomarker sequence of interest and measurement protocol information used in a multiplex panel (sequence information input step), to input the learned error characteristic obtained by the learning method according to any one of the eighth to tenth aspects and metadata associated with the error characteristic (learning result input step), to calculate a similarity degree between a biomarker sequence previously included in calibration data and a new biomarker sequence, using a measurement criterion for calculating a measure of similarity between two gene sequences (similarity degree calculation step), and to predict an error characteristic in a case of measuring a biomarker sequence that is not included in the calibration data, using the calculated similarity degree in combination with other related inputs and the learned error characteristic (error characteristic prediction step). The thirteenth aspect defines the prediction method corresponding to the sixth aspect.

According to a fourteenth aspect, the prediction method according to the thirteenth aspect may further comprise causing the processor to acquire a biomarker sequence that is most similar to the biomarker sequence not included in the calibration data and that has been available in the calibration data, using the predicted error characteristic (sequence acquisition step) and to reflect information of the acquired biomarker sequence in the determination of the biomarker sequence set in the determination method according to the eleventh or twelfth aspect (information reflection step). The fourteenth aspect defines the prediction method corresponding to the seventh aspect.

In addition, the scope of the present invention also includes programs (a learning program, a determination program, and a prediction program) that cause a processor to execute the learning method, the determination method, and the prediction method according to the above-described aspects and a non-transitory recording medium on which computer-readable codes of the programs are recorded.

As described above, the learning system, the determination system, the prediction system, the learning method, the determination method, and the prediction method according to the embodiments of the present invention have the following effects.
(1) A plurality of gene sequences can be measured together to handle a multiplex panel.
(2) It is possible to process a sample subjected to bisulfite conversion.
(3) It is possible to predict the measurement error using the sequence parameter and the protocol parameter as inputs.
(4) It is possible to determine whether or not to use the sequence for the purpose of analysis/classification.

### BRIEF DESCRIPTION OF THE DRAWINGS

Fig. 1 is a diagram illustrating an aspect in which DNA methylation is measured.
Fig. 2 is a diagram illustrating an aspect in which calibration data is created.
Fig. 3 is a diagram illustrating a learning system and data related to the learning system.
Fig. 4 is a diagram illustrating a configuration of the learning system.
Fig. 5 is a diagram illustrating an embodiment of a probability model.
Fig. 6 is a diagram illustrating a relationship between a determination system and a prediction system.
Fig. 7 is a diagram illustrating a configuration of the determination system.
Fig. 8 is a diagram illustrating a configuration of the prediction system.

### DESCRIPTION OF THE PREFERRED EMBODIMENTS

Hereinafter, embodiments of the present invention will be described. In the description, the accompanying drawings will be referred to as necessary. In addition, in the accompanying drawings, the illustration of some components may be omitted for convenience of explanation.

### [Creation of Calibration Data]

In the present invention, as illustrated in Fig. 2, first, it is necessary to create calibration data composed of important measurement protocol variables, such as a PCR temperature and the number of PCR cycles, from a blood sample 10 according to a wet experiment protocol 20. Preferably, the calibration data is designed such that appropriate data can be acquired for important variables. Finally, measurement results of sequences are stored in a calibration data database (DB) 30 together with protocol information (hereinafter, the database is referred to as a "DB" in some cases). In addition, in Fig. 2, a portion of a calibration data creation procedure is omitted for the sake of clarification.

A learning algorithm (a learning system and a learning method) uses the calibration data to learn a relationship between the protocol variables and the measurement characteristics thereof. Then, this system (a prediction system and a prediction method) can predict measurement error characteristics of a given biomarker sequence for a set of given measurement protocol variables (not included in the calibration data). The system (a determination system and a determination method) can determine whether or not the biomarker sequence is suitable for use in any quantitative research, using this prediction. Finally, even for a biomarker sequence that is not present in the calibration data, the system (the determination system and the determination method) according to the embodiment of the present invention can find the most similar sequence, for which the measurement error characteristics are known, and make a similar determination for a new sequence using the most similar sequence.

Specifically, in the present invention, the operation of a probability model is described in detail, and a "template-to-product" ratio is estimated to characterize the measurement error of the biomarker sequence. The "template" refers to the initial amount of the biomarker sequence (the amount before PCR amplification), and the "product" refers to the final amount of the same biomarker sequence after PCR amplification (the amount after PCR amplification).

### [Configuration of Learning System]

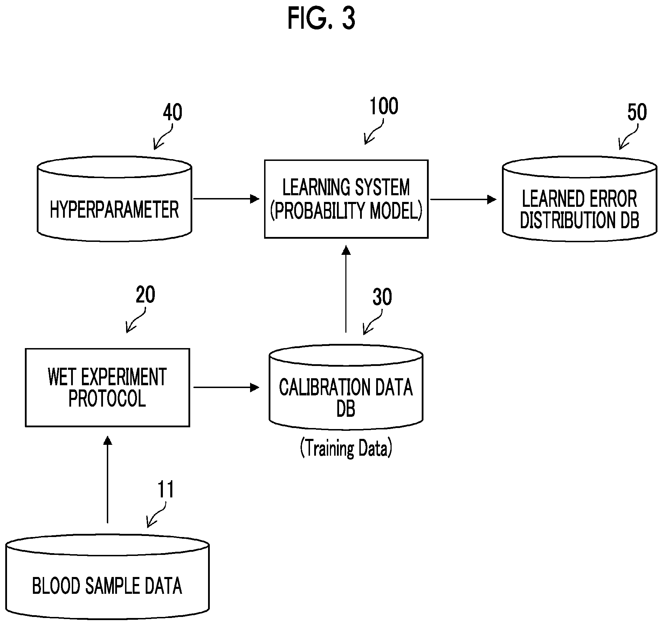

Fig. 3 illustrates a learning system 100 according to an aspect of the present invention, data related to the learning system 100, and the like. The application of this learning system for learning DNA methylation measurement error characteristics for a multiplex bisulfite PCR protocol is a minimum requirement for ensuring the novelty of the present invention. In addition, the learning system 100 may be accompanied by a determination system 200 (determination system) and a prediction system 300 (prediction system) that use the learning result (learned error distribution DB 50), which will be described below.

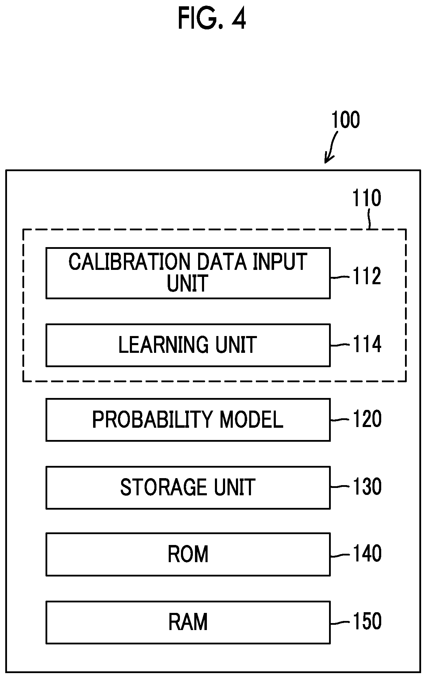

Fig. 4 is a diagram illustrating an example of a configuration of the learning system 100. As illustrated in Fig. 4, the learning system 100 comprises a processor 110 (a processor or a computer), a probability model 120 (probability model), a storage unit 130, a read only memory (ROM) 140, and a random access memory (RAM) 150. The processor 110 performs the overall control of processes performed by each unit of the learning system 100 and includes a calibration data input unit 112 and a learning unit 114.

The processor 110 may include a display control unit, a communication control unit, an output control unit, and the like (which are not illustrated) in addition to the elements illustrated in Fig. 4.

The processor 110 is configured by, for example, various processors, such as a central processing unit (CPU), a graphics processing unit (GPU), a field programmable gate array (FPGA), and a programmable logic device (PLD), and electric circuits. In a case where these processors and electric circuits execute software (program), codes readable by a computer (for example, various processors and electric circuits constituting the processor and/or combinations thereof) in the software to be executed are stored in a non-transitory and tangible recording medium, such as the ROM 140, and the computer refers to the software. The software that is stored in the non-transitory and tangible recording medium includes programs (a learning program, a prediction program, and a determination program) for executing the learning method, the prediction method, and the determination method according to the embodiments of the present invention and data used during the execution. The codes may be recorded on various non-transitory and tangible recording media, such as a magneto-optical recording device and a semiconductor memory, instead of the ROM 140. In a case of the processes using the software, for example, the RAM 150 is used as a transitory storage area. In addition, for example, the data stored in the non-transitory and tangible recording medium (not illustrated), such as an electronically erasable and programmable read only memory (EEPROM) or a flash memory, can also be referred to. The storage unit 130 may be used as the "non-transitory and tangible recording medium".

The storage unit 130 is configured by various storage devices, such as a hard disk and a semiconductor memory, and a control unit thereof and can store the above-described calibration data, the execution conditions and execution results (data of the learned error distribution) of the learning method, and the like.

The learning system 100 may include a display device (for example, a liquid crystal monitor) and an operation device (for example, a mouse or a keyboard) which are not illustrated, in addition to the elements illustrated in Fig. 4. The calibration data, data of an error distribution, and the like can be displayed on the display device. In addition, the user can perform an operation necessary for executing the learning method (learning program) according to the embodiment of the present invention through the operation unit.

Fig. 3 illustrates blood sample data 11 which is any biological data including a tissue sample. As illustrated in Fig. 1, the blood sample data 11 is measured by a measurement procedure including the above-described STEP 1 and STEP 2 and DNA sequence determination and has some variables (important variables), such as the number of PCR cycles, affecting the effectiveness of the blood sample data 11. It is necessary to obtain data from the values of some of the variables. Therefore, the relevant variables are identified first, and measurement is performed within the range of these values. For example, in a case where the number of PCR cycles is the only important variable, it is possible to generate data of the same blood samples with 5, 10, and 15 PCR cycles. This is so-called calibration data.

### [Probability Model]

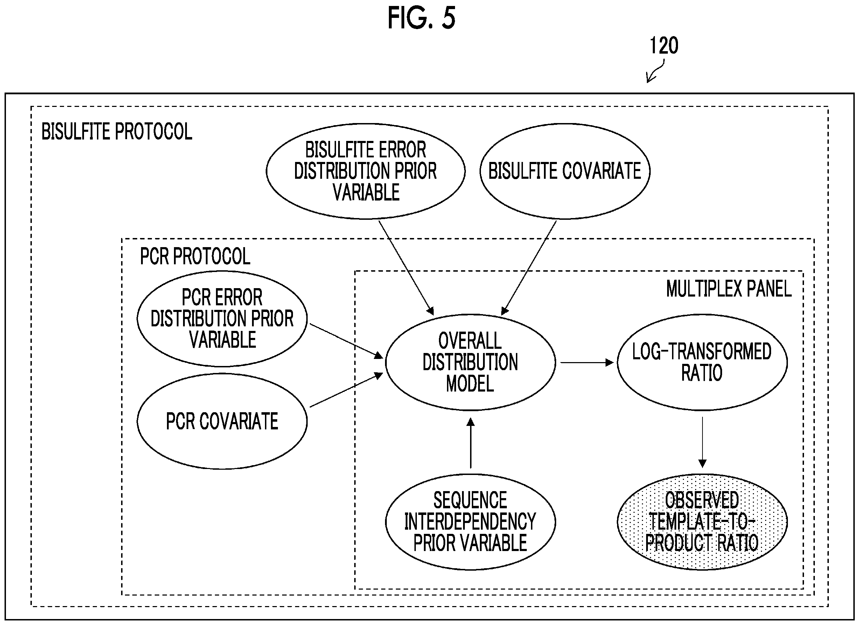

The learning system 100 trains the probability model using the calibration data (training data) stored in the calibration data DB 30 (calibration data input step) (learning step). Fig. 5 illustrates a probability model 120, which is an example of the probability model, through a Bayesian hierarchical model. The important novelty of the present invention is to use (i) prior information of a bisulfite conversion error (prior parameter; the same applies hereinafter), (ii) prior information of a covariate of bisulfite conversion, and (iii) prior information of interdependency of amplification of the biomarker sequence. These prior information items (i) to (iii) correspond to first to third parameters according to the embodiment of the present invention and thus correspond to the above-described problems 1 to 3. In a case where the above-described three elements are integrated, the present invention is different from the model according to the related art such as JP2017-523437A and "Measuring and Mitigating PCR Bias in Microbiome Data", Justin D. Silverman et al., [Searched on March 22, 2022], Internet (https://www.biorxiv.org/content/10.1101/604025v1).

In addition, it is possible to solve an integrated problem of the problem 1 + the problem 2 + the problem 3, using the three factors. The learning system 100 is adjusted through a series of hyperparameters (hyperparameters 40) according to an optimization method (for example, a loss function for minimization). This adjustment is performed by checking the final performance of the system and selecting hyperparameters that maximize the final performance.

In addition, the first to third parameters are a portion of the probability model 120 (therefore, a portion of the learning system 100), and the values of these parameters are updated during a training process. Further, since the first to third parameters are a portion of the learning system 100, the first to third parameters are not illustrated in Fig. 3.

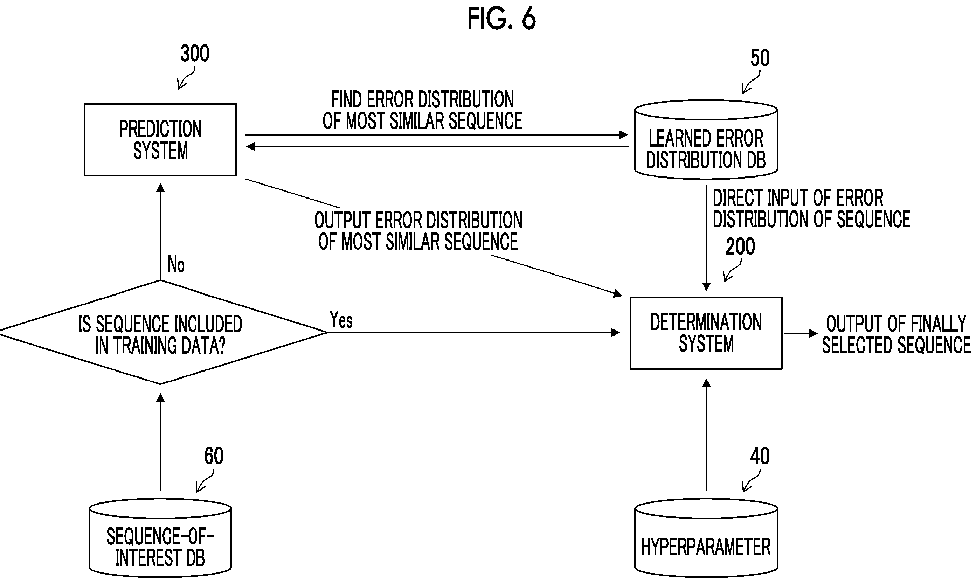

On the other hand, in a case in which the hyperparameters are used, it is possible to control a certain aspect of the probability model 120. However, the values of the hyperparameters are set by the user and are not updated during the training process. In addition, the hyperparameters are different between the learning system 100 and the determination system 200 (see Fig. 6).

More specifically, for a biomarker of a binomial distribution, it is possible to select modeling the bisulfite conversion error. Therefore, the prior probability of the bisulfite conversion error (an example of the prior parameter) can be selected as a value between [0, 1]. In a case where the prior probability is 0, complete conversion (100% conversion between Cu and uracil and 0% conversion of Cm) of the biomarker is assumed. In a case where the prior probability is greater than 0, incomplete conversion (only a portion of Cu is converted into uracil and a portion of Cm is also converted into uracil) of the biomarker is assumed. Ideally, the prior variable needs to be set from empirical data analysis. The bisulfite covariate includes the amount of sulfite added to the sample measured in nanograms and the initial amount of DNA. In this way, the bisulfite conversion error initialized with the prior probability is the first parameter.

Similarly, a PCR error distribution can be modeled by a multinomial distribution, and an appropriate prior probability can be set. In this stage, a sequence count (the number of sequences) after PCR can be represented by N1, N2, ..., and Nx in a case where the number of selected biomarkers is x, and the sequence count can be modeled as the multinomial distribution. Here, "Ni" is the sequence count of an i-th biomarker. A PCR covariate may include a factor selected for creating calibration data, such as a PCR temperature or the number of PCR cycles.

The novelty of the present invention is the ability to consider the possibility of two different counts from the same sequence. One count is the count of the basifications of a certain sequence after bisulfite conversion (the count of methylated sequences), and the other count is the count of debasification types of the sequences (the count of unmethylated sequences). As a result, it is considered that the number of possibilities, such as N1_M, N1_U, N2_M, and N2_U, is doubled. Here, "Ni_M" indicates the count of the methylated sequences for the i-th biomarker, and "Ni_U" indicates the count of the unmethylated sequences for the i-th biomarker. Since Nx_M and Nx_U impose natural constraints on each other (the fact that one average count is large means that the other average count is small), it is possible to simplify the modeling problem using the constraints (interdependency). Therefore, the interdependency of the amplification of the biomarker sequences initialized by the prior probability is a second parameter.

Finally, the overall distribution model (a model indicating the bias of the entire PCR) quantifies the sequences of all of the biomarkers, that is, the total number of N1 + N2 + ... + Nx and can even be used to impose interdependency constraints (configuration data constraints) through the biomarker counts (for example, in a case where N1 is too high, N3 is too low). Each of N1, N2, and the like (each count) is a multinomial distribution. Therefore, it is considered that, under conditions that the number of selected biomarkers is large (for example, 30 or more), the sum of the counts (the sum of the individual counts calculated by the multinomial distribution) follows a Gaussian distribution to satisfy the central limit theorem. The interdependency (configuration data constraints) between the sequence types may not be immediately clear. However, it is known that the interdependency is present in a case where a plurality of sequences (a plurality of biomarker sequences) are simultaneously amplified using a universal primer. Therefore, the bias of the entire PCR initialized by the prior probability is a third parameter.

The universal primer can be used only after appropriate adapter sequences are deployed at both ends of a target biomarker sequence. The limited amount of the universal primer added in this stage creates compositional dependence between the biomarker sequences and affects pure signal amplification. The second novelty of the present invention is formed by imposing the configuration data constraints on the multiplex panel during PCR amplification and modeling the relative abundance of the biomarker sequences using the universal primer.

### [Positioning of Determination System and Prediction System]

As illustrated in Fig. 6, the learning system 100 may be accompanied by the determination system 200 (determination system) and the prediction system 300 (prediction system). It is recommended to add the determination system and the prediction system to the learning system 100 as an option. The addition of the determination system 200 and the prediction system 300 makes it possible for the determination system 200 to find the best subset of candidate biomarkers using, for example, the error characteristics learned by the learning system 100 (by executing the determination method according to the embodiment of the present invention including a learning result input step, a score input step, a subset selection step, and the like). Therefore, it is possible to give information to a selection criterion for the biomarker sequence (by executing the prediction method according to the embodiment of the present invention including an information reflection step and the like; the prediction system 300) to help to effectively utilize the learning system 100.

The learning system 100 comprises the probability model 120 that maximizes or minimizes an optimization criterion using a statistical means to learn the optimization criterion, and this learning widely covers the meaning of "training" an algorithm. On the other hand, the determination system 200 functions after the learning system 100 ends the learning. Since the determination system 200 does not have a defined optimization criterion for maximization or minimization, the "training" is not performed, and the system is not trained. In addition, the determination system 200 is configured to include hyperparameters for making the system "adjustable".

### [Configuration of Determination System and Prediction System]

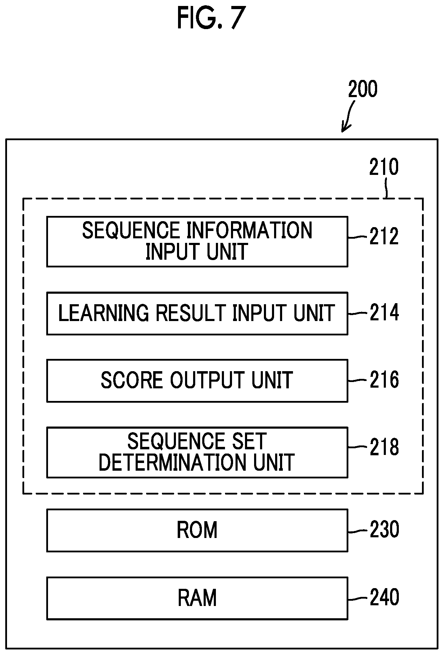

Fig. 7 is a diagram illustrating a configuration of the determination system 200. As illustrated in Fig. 7, the determination system 200 comprises a processor 210 (processor), a ROM 230 (non-transitory and tangible recording medium), and a RAM 240. The processor 210 comprises a sequence information input unit 212, a learning result input unit 214, a score output unit 216, and a sequence set determination unit 218. In addition to these elements, the determination system 200 may include a display control unit, a display device, a storage device, an operation unit, and the like which are not illustrated.

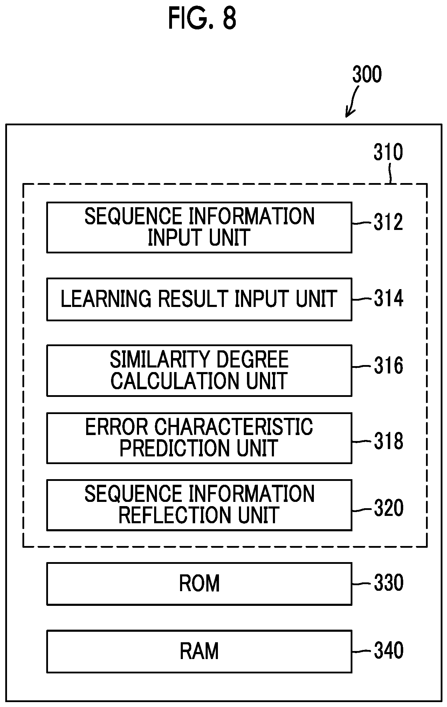

Fig. 8 is a diagram illustrating a configuration of the prediction system 300. As illustrated in Fig. 8, the prediction system 300 comprises a processor 310 (processor), a ROM 330 (non-transitory and tangible recording medium), and a RAM 340. The processor 310 comprises a sequence information input unit 312, a learning result input unit 314, a similarity degree calculation unit 316, an error characteristic prediction unit 318, and a sequence information reflection unit 320. In addition to these elements, the prediction system 300 may include a display control unit, a display device, a storage device, an operation unit, and the like which are not illustrated.

The elements of the determination system 200 and the prediction system 300 are configured by various processors, such as a CPU, a GPU, an FPGA, and a PLD, and electric circuits similarly to the learning system 100. In a case where these processors and electric circuits execute software (program), codes readable by the computer in the software to be executed are stored in a non-transitory and tangible recording medium, such as the ROM 230 or the ROM 330, and the computer refers to the software. The software that is stored in the non-transitory and tangible recording medium includes programs (the prediction program and the determination program) for executing the prediction method and the determination method according to the embodiments of the present invention and data used during the execution. The codes may be recorded on various non-transitory and tangible recording media, such as a magneto-optical recording device and a semiconductor memory, instead of the ROM 230 or the ROM 330. In a case of the processes using the software, for example, the RAM 240 or the RAM 340 is used as a transitory storage area. In addition, for example, the data stored in the non-transitory and tangible recording medium (not illustrated), such as an EEPROM or a flash memory, can also be referred to.

### [Determination of Biomarker Sequence Set Based on Score]

Hereinafter, a binary-based approach and a combination-based approach, which are two rough classifications of a "score optimization process (optimization method)", will be described.

In a binary-based optimization criterion, the sequence information input unit 212 (processor) of the determination system 200 inputs a nucleotide sequence of a biomarker sequence of interest and measurement protocol information (sequence information input step), and the learning result input unit 214 (processor) inputs the learned error characteristics and metadata associated with the error characteristics from the learning system 100 (learning result input step). The score output unit 216 (processor) can assign, for example, a score of {+1, 0, -1} (measurement error score; an example of a first score) to each biomarker sequence on the basis of the inclination of a measurement error graph generated as a result, independently considering the learned measurement error characteristics (score output step). The sequence set determination unit 218 can sum up the scores (first scores) from the order of each biomarker and determine whether or not to use a combination of the biomarker sequences (biomarker sequence set) (sequence set determination step).

In a case where this is more robustly implemented, the combination-based optimization criterion can be designed by the same method as that in the feature selection algorithm. The feature selection algorithm depends on the output of the quantitative model and updates the criteria in order to optimize the performance of the quantitative model. The view of the feature selection algorithm according to the related art is modified to use the same score (first score) as the signal in order to give the best information for the selection of a subset from a given biomarker sequence set in consideration of the score (first score) resulting from the measurement error characteristics. Therefore, it is possible to design the combination-based optimization criterion used in the present invention. Even in a case of the combination-based optimization criterion, it is possible to determine the biomarker sequence set using each element of the determination system 200 as in a case of the binary-based optimization criterion (the execution of the sequence information input step to the sequence set determination step).

In addition, in the above-described binary-based optimization criterion, the score is independently assigned to each biomarker sequence. However, in the case of the combination-based optimization criterion, the score is assigned to a combination of the biomarker sequences. Therefore, in a case where the smallness of the measurement error may be independently treated for each marker sequence, the binary-based optimization criterion is suitable. In a case where the interdependency is particularly large, the combination-based optimization criterion is suitable. The interdependency is, for example, a case where "the measurement error is small in a case where biomarker sequence 1 is measured at the same time as biomarker sequence 2, but is large in a case where biomarker sequence 1 is measured at the same time as biomarker sequence 3".

In the present invention, not only the above-described measurement error score (first score) but also a "score that is higher as the degree of association with a disease to be predicted is larger" (a degree-of-association score; an example of a second score) can be considered, and the balance between these scores can be optimized to determine the biomarker sequence set. In a case where the degree-of-association score (second score) is used in combination, the biomarker sequence set can be determined by optimizing the balance between the above-mentioned measurement error score (first score) and the degree-of-association score (second score) (for example, maximizing the arithmetic mean or geometric mean of the measurement error score and the degree-of-association score). In this case, similarly to the measurement error score, the degree-of-association score can be assigned independently to each biomarker sequence or can be assigned to a combination of the biomarker sequences. For example, in a case where all of markers 1, 2, and 3 are related to a disease, the correlation between the markers 1 and 2 is small, and the correlation between the markers 1 and 3 is large, a combination of the markers 1 and 2 is more effective for disease prediction and has a higher degree-of-association score.

Which optimization criterion (the binary-based optimization criterion, the feature selection algorithm, or the combination-based optimization criterion) is best depends on the application field, the user, and the time constraint, and the optimization criterion can be appropriately selected according to those conditions. This output is a set of biomarker sequences that the system considers together, and there is a minimum error in the measurement error protocol given for multiplex PCR sequence determination. The embodiment can be changed on the basis of the implementation of the determination system (regardless of whether or not to consider the balanced sequence selection), in order to consider the fifth and twelfth aspects of the present invention.

In addition, the determination system 200 depends on the error distribution (in Fig. 6, the learned error distribution database 50) obtained by the learning system 100 and is not capable of calculating the score of the biomarker sequence that is not included in the original calibration data. The prediction system 300 according to the sixth and seventh aspects of the present invention (and the prediction method according to the thirteenth and fourteenth aspects of the present invention) is required for the prediction of the measurement error characteristics of the biomarker sequence that is not included in the calibration data, as illustrated in Fig. 6 and as described below. The prediction system 300 is an addition to the learning system 100 (learning system) according to the embodiment of the present invention as described above for the determination system 200 and depends on the new use case, presence, and importance of the biomarker sequence.

### [Prediction of Measurement Error Characteristics of Biomarker Sequence Not Included in Calibration Data]

In a case where it is determined that the biomarker sequence of interest included in a sequence-of-interest database 60 is the biomarker sequence that is not included in the calibration data (in a case where the result of the determination of "Is the sequence included in the training data?" in Fig. 6 is YES), a method for predicting the measurement error characteristics of the biomarker sequence of interest will be described. In this case, first, the input is transmitted to the prediction system 300. Specifically, the sequence information input unit 312 (processor) of the prediction system 300 inputs the nucleotide sequence of the biomarker sequence of interest and the measurement protocol information (sequence information input step), and the learning result input unit 314 (processor) inputs, for example, the learned error characteristics and the metadata associated with the error characteristics from the learning system 100 (learning result input step). Here, the "metadata" is, for example, the type of a gene (a promoter or an enhancer) and a region of the gene (a transcription start site or the like), but is not limited thereto.

Then, the similarity degree calculation unit 316 (processor) calculates a similarity degree between a biomarker sequence (a biomarker sequence that has been available in the calibration data) previously included in the calibration data and a new biomarker sequence (biomarker sequence of interest), using a measurement criterion (scale of similarity) for the similarity degree between two gene sequences, such as a Levenshtein distance or GC content (the ratio of guanine to cytosine among nitrogen bases in a DNA molecule) (similarity degree calculation step). The similarity degree calculation unit 316 detects a biomarker sequence that is "most similar" to the biomarker sequence of interest from the biomarker sequences present in the learned error distribution database 50 (similarity degree calculation step). The prediction system 300 can acquire the learned error characteristics corresponding to "the most similar sequence" using the information of the detected "most similar sequence" (from the learned error distribution database 50) (an error characteristic prediction step and a sequence acquisition step). Therefore, it is possible to completely implement the sixth and thirteenth aspects of the present invention. The sequence information reflection unit 320 can also reflect this information in the determination of the biomarker sequence set in the determination system 200 in combination with the determination system 200 implementing the fourth and fifth aspects of the present invention (and the determination method according to the eleventh and twelfth aspects of the present invention) (information reflection step).

### [Examples]

It is considered that a candidate set for a gene sequence first shows a sufficient change in a measurement-related factor such as GC content. For example, assuming that only sequence GC content is important, a 3-gene sequence with "high" GC content and a 3-gene sequence with "low" GC content can be determined for simultaneous measurement. Then, a series of important measurement protocol-related variables is specified, and a range is considered. Then, the wet experiment procedure is executed for the entire range in which all values and all variables are considered. For example, in a case where a PCR cycle of {5, 10, 15} is considered and the methylation ratio is considered to be {5%, 10%}, six independent measurements (3 * 2 = 6) of six genes are performed from the aliquots of the same biological specimen. Then, the above-described probability model used in the learning system was trained, and the hyperparameters of the determination system were adjusted (tuned). However, as in the case of cancer diagnosis, a determination model can be used to evaluate whether the gene is good or bad for the measurement characteristics while searching for more gene biomarkers.

Considering that an artificial intelligence cancer classification model trained by the measurement of 100 gene sequences is performed with a sensitivity of 70%, some of the reasons for low performance may be a large amount of measurement noise in some genes. In a case where 100 genes are reconsidered using the above-described system and the genes that are difficult to measure are removed, the performance of artificial intelligence is likely to increase to 80% by avoiding the measurement error, and thus the system has higher robustness.

The embodiments described above have the following effects.
(1) A plurality of gene sequences can be measured together to handle a multiplex panel.
(2) It is possible to process a sample subjected to bisulfite conversion.
(3) It is possible to predict the measurement error using the sequence parameter and the protocol parameter as inputs.
(4) It is possible to determine whether or not to use the sequence for the purpose of analysis/classification.
(5) It is possible to perform the analysis, diagnosis, or the like using the biomarker sequence (for example, the described-above classification of cancer by AI) with high accuracy, on the basis of the appropriately learned error characteristics, the appropriately selected biomarker sequence set (and the subset thereof), and the measurement error of the biomarker sequence set predicted with high accuracy.

The embodiments of the present invention have been described above. However, the present invention is not limited to the above-described aspects and can be modified in various ways.

### Explanation of References

10: blood sample
11: blood sample data
20: wet experiment protocol
30: calibration data DB
40: hyperparameter
50: learned error distribution database
60: sequence-of-interest database
100: learning system
110: processor
112: calibration data input unit
114: learning unit
120: probability model
130: storage unit
200: determination system
210: processor
212: sequence information input unit
214: learning result input unit
216: score output unit
218: sequence set determination unit
300: prediction system
310: processor
312: sequence information input unit
314: learning result input unit
316: similarity degree calculation unit
318: error characteristic prediction unit
320: sequence information reflection unit

## Claims

1. A learning system that learns a relationship between a measurement protocol variable and an error characteristic occurring as a result of a biomarker sequence, the learning system comprising:
a processor configured to:
input calibration data that is designed such that appropriate data is capable of being acquired for an important variable; and
learn a characteristic of an error distribution over each measurement protocol for the important variable, using a probability model,
wherein the probability model includes a first parameter that is initialized with an appropriately selected prior parameter in order to model an error of bisulfite conversion, a second parameter that is initialized with an appropriately selected prior parameter in order to model interdependency of amplification of the biomarker sequence, and a third parameter that is initialized with an appropriately selected prior parameter in order to model a bias of an entire PCR.

2. The learning system according to claim 1,
wherein the second parameter is a parameter that is obtained by separately acquiring counts of methylated sequences and unmethylated sequences of genes after the bisulfite conversion and modeling the acquired counts with a multinomial distribution capable of separately determining a prior variable for each of the methylated sequences and the unmethylated sequences.

3. The learning system according to claim 1 or 2,
wherein the third parameter is a parameter subjected to a configuration data constraint in which a sum of individual counts calculated by a multinomial distribution follows a Gaussian distribution in a case where a plurality of sequences are simultaneously amplified using a universal primer.

4. A determination system comprising:
a processor configured to:
input a nucleotide sequence of a biomarker sequence of interest and measurement protocol information used in a multiplex panel;
input the learned error characteristic and metadata associated with the error characteristic from the learning system according to any one of claims 1 to 3;
output a first score for a set of possible biomarker sequences using the input nucleotide sequence, measurement protocol information, learned error characteristic, and metadata, according to a predetermined criterion; and
determine a biomarker sequence set in consideration of a value of the first score for each set.

5. The determination system according to claim 4,
wherein the processor is configured to:
input a second score for each biomarker sequence to be determined; and
optimize a balance between the first score and the second score in consideration of the first score for each biomarker sequence in the biomarker sequence set to select a best subset of the multiplex panel.

6. A prediction system that predicts a measurement error characteristic of a gene sequence, the prediction system comprising:
a processor configured to:
input a nucleotide sequence of a biomarker sequence of interest and measurement protocol information used in a multiplex panel;
input the learned error characteristic and metadata associated with the error characteristic from the learning system according to any one of claims 1 to 3;
calculate a similarity degree between a biomarker sequence previously included in calibration data and a new biomarker sequence, using a measurement criterion for calculating a measure of similarity between two gene sequences; and
predict an error characteristic in a case of measuring a biomarker sequence that is not included in the calibration data, using the calculated similarity degree in combination with other related inputs and the learned error characteristic.

7. The prediction system according to claim 6,
wherein the processor is configured to:
acquire a biomarker sequence that is most similar to the biomarker sequence not included in the calibration data and that has been available in the calibration data, using the predicted error characteristic; and
reflect information of the acquired biomarker sequence in the determination of the biomarker sequence set in the determination system according to claim 4 or 5.

8. A learning method executed by a learning system that includes a processor and learns a relationship between a measurement protocol variable and an error characteristic occurring as a result of a biomarker sequence, the learning method comprising:
causing the processor to input calibration data that is designed such that appropriate data is capable of being acquired for an important variable and to learn a characteristic of an error distribution over each measurement protocol for the important variable, using a probability model,
wherein the probability model includes a first parameter that is initialized with an appropriately selected prior parameter in order to model an error of bisulfite conversion, a second parameter that is initialized with an appropriately selected prior parameter in order to model interdependency of amplification of the biomarker sequence, and a third parameter that is initialized with an appropriately selected prior parameter in order to model a bias of an entire PCR.

9. The learning method according to claim 8,
wherein the second parameter is a parameter that is obtained by separately acquiring counts of methylated sequences and unmethylated sequences of genes after the bisulfite conversion and modeling the acquired counts with a multinomial distribution capable of separately determining a prior variable for each of the methylated sequences and the unmethylated sequences.

10. The learning method according to claim 8 or 9, wherein the third parameter is a parameter subjected to a configuration data constraint in which a sum of individual counts calculated by a multinomial distribution follows a Gaussian distribution in a case where a plurality of sequences are simultaneously amplified using a universal primer.

11. A determination method executed by a determination system including a processor, the determination method comprising:
causing the processor to input a nucleotide sequence of a biomarker sequence of interest and measurement protocol information used in a multiplex panel, to input the learned error characteristic obtained as a result of the learning method according to any one of claims 8 to 10 and metadata associated with the error characteristic, to output a first score for a set of possible biomarker sequences using the input nucleotide sequence, measurement protocol information, learned error characteristic, and metadata, according to a predetermined criterion, and to determine a biomarker sequence set in consideration of a value of the first score for each set.

12. The determination method according to claim 11, further comprising:
causing the processor to input a second score for each biomarker sequence to be determined and to optimize a balance between the first score and the second score in consideration of the first score for each biomarker sequence in the biomarker sequence set to select a best subset of the multiplex panel.

13. A prediction method executed by a prediction system that includes a processor and that predicts a measurement error characteristic of a gene sequence, the prediction method comprising:
causing the processor to input a nucleotide sequence of a biomarker sequence of interest and measurement protocol information used in a multiplex panel, to input the learned error characteristic obtained by the learning method according to any one of claims 8 to 10 and metadata associated with the error characteristic, to calculate a similarity degree between a biomarker sequence previously included in calibration data and a new biomarker sequence, using a measurement criterion for calculating a measure of similarity between two gene sequences, and to predict an error characteristic in a case of measuring a biomarker sequence that is not included in the calibration data, using the calculated similarity degree in combination with other related inputs and the learned error characteristic.

14. The prediction method according to claim 13, further comprising:
causing the processor to acquire a biomarker sequence that is most similar to the biomarker sequence not included in the calibration data and that has been available in the calibration data, using the predicted error characteristic, and to reflect information of the acquired biomarker sequence in the determination of the biomarker sequence set in the determination method according to claim 11 or 12.
